# EUROPÄISCHE PATENTSCHRIFT

(11) **EP 2 976 059 B1**
(45) Veröffentlichungstag und Bekanntmachung des Hinweises auf die Patenterteilung: **31.01.2018**
(21) Anmeldenummer: 14702268.5
(22) Anmeldetag: 03.02.2014
(51) Int. Cl.: A61K 8/39, A61Q 5/06, A61K 8/86

(54) **MITTEL ZUR TEMPORÄREN VERFORMUNG KERATINHALTIGER FASERN**
COMPOSITION FOR TEMPORARILY SHAPING KERATINIC FIBERS
COMPOSITION POUR LA FORMATION TEMPORAIRE DES FIBRES CERATINIQUES

(30) Priorität: 19.03.2013 DE 102013204807
(43) Veröffentlichungstag der Anmeldung: 27.01.2016
(73) Patentinhaber: Henkel AG & Co. KGaA, 40589 Düsseldorf (DE)
(72) Erfinder: KNAPPE, Thorsten, 22869 Schenefeld (DE); LANGE, Julia Bibiane, 24641 Sievershütten (DE)
(86) Internationale Anmeldenummer: PCT/EP2014/052040
(87) Internationale Veröffentlichungsnummer: WO 2014/146819

(56) Entgegenhaltungen:
- EP-A1- 0 867 167
- EP-A1- 1 800 648
- EP-A2- 1 559 395
- EP-A2- 1 997 372

## Beschreibung

Die Anmeldung beschreibt kosmetische Zusammensetzungen zur temporären Verformung keratinischer Fasern, insbesondere menschlicher Haare.

Stylingmittel zur Verformung keratinhaltiger Fasern sind lange bekannt und finden in verschiedener Ausgestaltung Einsatz zum Aufbau, zur Auffrischung und zur Fixierung von Frisuren, die sich bei vielen Haartypen nur unter Verwendung festigender Wirkstoffe erhalten lassen. Dabei spielen sowohl Haarbehandlungsmittel, die einer permanenten, als auch solche, die einer temporären Formgebung der Haare dienen, eine wichtige Rolle.

Die Mittel zur temporären Formgebung liegen in verschiedenen Konfektionsformen vor, wobei bei den Treibgas-freien Mitteln insbesondere Haarwachse und Haargele verbreitet sind. Diese Mittel unterscheiden sich aus Verbrauchersicht hinsichtlich ihrer Produktoptik und Produkthaptik. Die Optik und die Haptik des Haarkosmetikums ist neben weiteren sensorischen Eigenschaften wie dem Geruch ein wesentliches Mittel zur Unterstützung Produkt- oder Markenversprechens und damit mitentscheidend für den kommerziellen Erfolg des Produktes. Aus diesem Grund sind Optik und Haptik des Stylingmittels in der Regel nicht das zufällige Ergebnis der Kombination spezifischer Wirkstoffe sondern das Resultat einer gezielten Produktentwicklung.

Um kosmetische Mittel mit erwünschten sensorischen Eigenschaften zu erhalten, werden diesen häufig Emulgatoren zugesetzt. Dies gilt insbesondere im Falle gelförmiger, insbesondere transparenter, gelförmiger Haarstylingmittel. Weite Verbreitung haben in diesem Zusammenhang Phosphatgruppen-haltige Emulgatoren wie beispielsweise Oleth-3 Phosphat gefunden. Der Einsatz dieser Phosphatgruppen-haltigen Emulgatoren kann jedoch zu Hautreizungen führen.

Die Aufgabe dieser Patentanmeldung war es daher, kosmetische Mittel zur temporären Verformung keratinischer Fasern mit verminderten Reizpotential zur Verfügung zur stellen, die sich sowohl durch eine außergewöhnliche Optik und Haptik als auch durch gute kosmetische Eigenschaften, insbesondere durch einen hohen Glanz der mit diesen Mitteln behandelten keratinischen Fasern, auszeichnen.

Es wurde festgestellt, dass sich diese Aufgaben durch die Kombination zweier spezifischer Anlagerungsprodukte von Polyethylenoxid an Fettalkohol lösen lassen, sofern dem kosmetischen Mittel zusätzlich Glycerin zugesetzt wird.

Haarstylingmittel, die neben weiteren Inhaltsstoffen Fettalkoholethoxylate und Glycerin enthalten, werden beispielsweise in der deutschen Offenlegungsschrift DE 10 2004 004 394 A1 (Wella) und der europäischen Patentanmeldung EP 1 800 648 A1 (Beiersdorf) beschrieben.

Ein erster Gegenstand der vorliegenden Anmeldung ist daher eine kosmetische Zusammensetzung zur temporären Verformung keratinhaltiger Fasern, enthaltend
a) 4,0 bis 17 Gew.-%mindestens eines Emulgators a) aus der Gruppe der ethoxylierten ungesättigten C16-18 Fettalkohole mit einem durchschnittlichen Ethoxylierungsgrad von 4 bis 6;
b) 6,0 bis 24 Gew.-% mindestens eines Emulgators b) aus der Gruppe der ethoxylierten gesättigten C16-18 Fettalkohole mit einem durchschnittlichen Ethoxylierungsgrad von 12 bis 30;
c) 6,0 bis 30 Gew.-% Glycerin.

Als "durchschnittlicher Ethoxylierungsgrad" wird dabei die Molmenge Ethylenoxid bezeichnet, mit der ein Mol Fettalkohol umgesetzt wurde. Der Ethoxylierungsgrad stellt also einen statistischen Mittelwert dar, die für ein spezielles Produkt einer ganzen oder einer gebrochenen Zahl entsprechen kann.

Ein erster wesentlicher Bestandteil erfindungsgemäßer kosmetischer Zusammensetzungen ist der Emulgator a) aus der Gruppe der ethoxylierten ungesättigten C16-18 Fettalkohole mit einem durchschnittlichen Ethoxylierungsgrad von 4 bis 6. Bevorzugt ist der Einsatz eines ethoxylierten Oleylalkohols als Emulgator a). Besonders bevorzugt werden insbesondere die ethoxylierten Fettalkohole mit der INCI-Bezeichnungen Oleth-4, Oleth-5 und Oleth-6. In bevorzugten kosmetischen Zusammensetzungen weist der Emulgator a) einen einen durchschnittlichen Ethoxilierungsgrad von 5 auf. Ein ganz besonders bevorzugter Emulgator a) ist der ethoxylierte Fettalkohol mit der INCI-Bezeichnung Oleth-5.

Durch den Gewichtsanteil des Emulgators a) am Gesamtgewicht der kosmetischen Zusammensetzung können deren kosmetische, optische und haptische Eigenschaften in vorteilhafter Weise beeinflusst werden. Besonders bevorzugte erfindungsgemäße kosmetische Zusammensetzungen sind dadurch gekennzeichnet, dass diese bezogen auf ihr Gesamtgewicht 6,0 bis 15 Gew.-%, vorzugsweise 7,0 bis 14 Gew.-% und insbesondere 8,0 bis 13 Gew.-% des Emulgators a) enthalten.

Als zweiten wesentlichen Bestandteil enthaltend die erfindungsgemäßen kosmetischen Zusammensetzungen einen Emulgator b) aus der Gruppe der ethoxylierten gesättigten C16-18 Fettalkohole mit einem durchschnittlichen Ethoxylierungsgrad von 12 bis 30. Bevorzugte Fettalkohole sind ausgewählt aus der Gruppe Cetylalkohol (Hexadecanol) und Stearylalkohol (Octadecanol). Die C16-18 Fettalkohole können sowohl allein als auch in Mischung eingesetzt werden. Besonders bevorzugt werden insbesondere die ethoxylierten Fettalkohole mit den INCI-Bezeichnungen Ceteth-15, Ceteth-16, Ceteth-20, Isoceteth-20, Ceteareth-12, Ceteareth-15, Ceteareth-20, Ceteareth-23, Ceteareth-25, Ceteareth-30, Steareth-16, Steareth-20, Steareth-21, Steareth-25 und Isosteareth-20, wobei die ethoxylierten Fettalkohole mit den INCI-Bezeichnungen Ceteth-20, Isoceteth-20, Ceteareth-20, Steareth-20 und Isosteareth-20 besonders bevorzugt werden. Kosmetische Zusammensetzung nach einem der vorherigen Ansprüche, dadurch gekennzeichnet, dass der Emulgator b) einen durchschnittlichen Ethoxilierungsgrad von 15 bis 25, vorzugsweise von 20 aufweist. Ein ganz besonders bevorzugter Emulgator b) ist der ethoxylierte Fettalkohol mit der INCI-Bezeichnung Ceteth-20.

Der Gewichtsanteil des Emulgators b) am Gesamtgewicht erfindungsgemäßer kosmetischer Zusammensetzungen beträgt vorzugsweise 8,0 bis 22 Gew.-%, bevorzugt 9,0 bis 20 Gew.-% und insbesondere 10 bis 18 Gew.-%. Diese Gewichtsanteile haben sich als vorteilhaft für die kosmetischen, optischen und haptischen Eigenschaften der erfindungsgemäßen Zusammensetzungen erwiesen.

Für die kosmetischen, optischen und haptischen Eigenschaften der erfindungsgemäßen Zusammensetzungen hat es sich als vorteilhaft erwiesen, die Emulgatoren a) und b) in spezifischen Gewichtsverhältnissen einzusetzten. Bevorzugte kosmetische Zusammensetzung sind daher dadurch gekennzeichnet, dass das Gewichtsverhältnis des Emulgators a) zum Emulgator b) 2:1 bis 1:5, vorzugsweise 1:1 bis 1:2 beträgt.

Als dritten wesentlichen Bestandteil enthalten die erfindungsgemäßen Zusammensetzungen Glycerin. Der Gewichtstanteil des Glycerins am Gesamtgewicht erfindungsgemäßer kosmetischer Zusammensetzungen beträgt vorzugsweise 10 bis 26 Gew.-%, bevorzugt 12 bis 20 Gew.-%.

Die erfindungsgemäßen kosmetischen Zusammensetzungen basieren vorzugsweise auf einem wässrig-alkoholischen Träger. Bevorzugt werden kosmetische Zusammensetzungen, die bezogen auf ihr Gesamtgewicht 20 bis 70 Gew.-%, vorzugsweise 30 bis 60 Gew.-% und insbesondere 40 bis 55 Gew.-% Wasser enthalten. Bevorzugte kosmetische Zusammensetzungen auf Grundlage wässrigalkoholischer Träger enthalten Wasser, Glycerin sowie mindestens einen Alkohol aus der Gruppe Ethanol, 1,3-Butylenglycol und Sorbitol. Bevorzugt ist der Einsatz mehrwertiger Alkohle aus der Gruppe 1,3-Butylenglycol und Sorbitol. Unabhängig von ihrem Wassergehalt sind solche kosmetischen Zusammensetzungen bevorzugt, die bezogen auf ihr Gesamtgewicht 0,1 bis 10 Gew.-%, vorzugsweise 0,5 bis 8,0 Gew.-% und insbesondere 1,0 bis 6,0 Gew.-% mindestens eines mehrwertigen Alkohols, vorzugsweise mindestens eines Alkohols aus der Gruppe 1,3-Butylenglycol und Sorbitol, enthält.

Bevorzugte kosmetische Zusammensetzungen enthalten als weiteren Bestandteil mindestens einen Fettstoff c). Bevorzugte kosmetische Zusammensetzungen sind dadurch gekennzeichnet, dass einen Fettstoffs c) aus der Gruppe der mineralischen, natürlichen und synthetischen Öle, insbesondere besonders bevorzugt aus der Gruppe der mineralischen Öle, enthalten.

Als natürliche (pflanzliche) Öle können Triglyceride und Mischungen von Triglyceriden eingesetzt werden. Bevorzugte natürliche Öle sind Kokosnussöl, (süßes) Mandelöl, Walnussöl, Pfirsichkernöl, Aprikosenkernöl, Avocadoöl, Teebaumöl (Tea Tree Oil), Sojaöl, Sesamöl, Sonnenblumenöl, Tsubakiöl, Nachtkerzenöl, Reiskleieöl, Palmkernöl, Mangokernöl, Wiesenschaumkrautöl, Distelöl, Macadamianussöl, Traubenkernöl, Amaranthsamenöl, Arganöl, Bambusöl, Olivenöl, Weizenkeimöl, Kürbiskernöl, Malvenöl, Haselnussöl, Safloröl, Canolaöl, Sasanquaöl, Jojobaöl, Rambutanöl, Kakaobutter und Shea-Butter.

Als synthetische Öle kommen Silikonverbindungen in Betracht. Geeignete Silikone können ausgewählt sein unter:
(i) Polyalkylsiloxanen, Polyarylsiloxanen, Polyalkylarylsiloxanen, die flüchtig oder nicht flüchtig, geradkettig, verzweigt oder cyclisch, vernetzt oder nicht vernetzt sind;
(ii) Polysiloxanen, die in ihrer allgemeinen Struktur eine oder mehrere organofunktionelle Gruppen enthalten, die ausgewählt sind unter:
   a) substituierten oder unsubstituierten aminierten Gruppen;
   b) (per)fluorierten Gruppen;
   c) Thiolgruppen;
   d) Carboxylatgruppen;
   e) hydroxylierten Gruppen;
   f) alkoxylierten Gruppen;
   g) Acyloxyalkylgruppen;
   h) amphoteren Gruppen;
   i) Bisulfitgruppen;
   j) Hydroxyacylaminogruppen;
   k) Carboxygruppen;
   l) Sulfonsäuregruppen; und
   m) Sulfat- oder Thiosulfatgruppen;
(iii) linearen Polysiloxan(A)- Polyoxyalkylen(B)- Blockcopolymeren vom Typ (A-B)ₙ mit n > 3;
(iv) gepfropften Siliconpolymeren mit nicht siliconhaltigem, organischen Grundgerüst, die aus einer organischen Hauptkette bestehen, welche aus organischen Monomeren gebildet wird, die kein Silicon enthalten, auf die in der Kette sowie gegebenenfalls an mindestens einem Kettenende mindestens ein Polysiloxanmakromer gepfropft wurde;
(v) gepfropften Siliconpolymeren mit Polysiloxan- Grundgerüst, auf das nicht siliconhaltige, organische Monomere gepfropft wurden, die eine Polysiloxan-Hauptkette aufweisen, auf die in der Kette sowie gegebenenfalls an mindestens einem ihrer Enden mindestens ein organisches Makromer gepfropft wurde, das kein Silicon enthält;
(vi) oder deren Gemischen.

Als mineralische Öle kommen insbesondere Mineralöle, Paraffin- und Isoparaffinöle sowie synthetische Kohlenwasserstoffe zum Einsatz. Besonders bevorzugt werden Paraffine, insbesondere flüssige Paraffine, vorzugsweise Paraffine mit der INCI Bezeichnung Paraffinum Liquidum. Der Gewichtsanteil des Fettstoffs c) am Gesamtgewicht erfindungsgemäßer kosmetischer Zusammensetzungen beträgt vorzugsweise 6,0 bis 22 Gew.-%, bevorzugt 8,0 bis 20 Gew.-% und insbesondere 10 bis 18 Gew.-%. Besonders bevorzugte kosmetische Zusammensetzungen sind dadurch gekennzeichnet, dass sie bezogen auf ihr Gesamtgewicht 6,0 bis 22 Gew.-%, vorzugsweise 8,0 bis 20 Gew.-% und insbesondere 10 bis 18 Gew.-% eines Fettstoffs c) aus der Gruppe der mineralischen, natürlichen und synthetischen Öle, insbesondere bevorzugt aus der Gruppe der mineralischen Öle, enthalten.

Die erfindungsgemäßen kosmetischen Zusammensetzungen können weitere Hilfs-, Pflege- und Zusatzstoffe enthalten. Der Gewichtsanteil der in den erfindungsgemäßen Zusammensetzungen neben den Komponenten a) bis d) enthaltenen weiteren Inhaltsstoffe, insbesondere der in diesen Zusammensetzungen enthaltenen weiteren Hilfs-, Pflege- und Zusatzstoffe am Gesamtgewicht der erfindungsgemäßen Zusammensetzungen beträgt vorzugsweise weniger als 10 Gew.-%, bevorzugt weniger als 7,0 Gew.-%, besonders bevorzugt weniger als 5,0 Gew.-% und insbesondere weniger als 3,0 Gew.-%. Der Gewichtsanteil dieser Hilfs-, Pflege- und Zusatzstoffe am Gesamtgewicht der erfindungsgemäßen kosmetischen Mittel kann beispielsweise 0,1 bis 5,0 Gew.-%, insbesondere 0,2 bis 3,0 Gew.-%, betragen.

Die erfindungsgemäßen Zusammensetzungen können eine oder mehrere filmbildende(s) Polymer(e) enthalten. Unter filmbildenden Polymeren sind solche Polymere zu verstehen, welche beim Trocknen einen kontinuierlichen Film auf dem Haar hinterlassen. Unter filmbildenden Polymeren werden weiterhin solche Polymere verstanden, die bei Anwendung in 0,01 bis 20 Gew.-%-iger wässriger, alkoholischer oder wässrigalkoholischer Lösung in der Lage sind, auf dem Haar einen transparenten Polymerfilm abzuscheiden.

Als filmbildende und/oder festigende Polymere eignen sich permanent als auch temporär kationische, anionische, nichtionische oder amphotere Polymere. Die filmbildenden Polymere können synthetischen oder natürlichen Ursprungs sein.

Geeignete und erfindungsgemäß bevorzugt eingesetzte synthetische, filmbildende, haarfestigende Polymere sind Homo- oder Copolymere, die aus mindestens einem der folgenden Monomere aufgebaut sind: Vinylpyrrolidon, Vinylcaprolactam, Vinylester wie z.B. Vinylacetat, Vinylalkohol, Acrylamid, Methacrylamid, C₁- bis C₇-Alkylacrylamid, C₁- bis C₇-Dialkylacrylamid, C₁- bis C₇-Alkylmethacrylamid, C₁- bis C₇-Dialkylmethacrylamid, C₁- bis C₇-Alkylacrylat, Acrylsäure, Propylenglykol, Ethylenglykol, wobei die C₁- bis C₇-Alkylgruppen dieser Monomere vorzugsweise C₁-bis C₃-Alkylgruppen sind. Beispielhaft seien genannt Homopolymere des Vinylcaprolactams, des Vinylpyrrolidons oder des N-Vinylformamids. Weitere geeignete synthetische filmbildende, haarfestigende Polymere sind z.B. Copolymerisate aus Vinylpyrrolidon und Vinylacetat, Vinylpyrrolidon und Styrol oder Terpolymere aus Vinylpyrrolidon, Vinylacetat und Vinylpropionat, Polyacrylamide. Geeignete natürliche filmbildende Polymere sind z.B. Cellulosederivate, z. B. Hydroxypropylcellulose mit einem Molekulargewicht von 30.000 bis 50.000 g/mol.

Weitere Beispiele für gebräuchliche filmbildende Polymere sind Acrylamide/Ammonium Acrylate Copolymer, Acrylamides/DMAPA Acrylates/Methoxy PEG Methacrylate Copolymer, Acrylamidopropyltrimonium Chloride/Acrylamide Copolymer, Acrylamidopropyltrimonium Chloride/Acrylates Copolymer, Acrylates/Acetoacetoxyethyl Methacrylate Copolymer, Acrylates/Acrylamide Copolymer, Acrylates/Ammonium Methacrylate Copolymer, Acrylates/t-Butylacrylamide Copolymer, Acrylates Copolymer, Acrylates/C1-2 Succinates/Hydroxyacrylates Copolymer, Acrylates/Lauryl Acrylate/Stearyl Acrylate/Ethylamine Oxide Methacrylate Copolymer, Acrylates/Octylacrylamide Copolymer, Acrylates/Octylacrylamide/Diphenyl Amodimethicone Copolymer, Acrylates/Stearyl Acrylate/Ethylamine Oxide Methacrylate Copolymer, Acrylates/VA Copolymer, Acrylates/VP Copolymer, Adipic Acid/Diethylenetriamine Copolymer, Adipic Acid/Dimethylaminohydroxypropyl Diethylenetriamine Copolymer, Adipic Acid/Epoxypropyl Diethylenetriamine Copolymer, Adipic Acid/Isophthalic Acid/Neopentyl Glycol/Trimethylolpropane Copolymer, Allyl Stearate/VA Copolymer, Aminoethylacrylate Phosphate/Acrylates Copolymer, Aminoethylpropanediol-Acrylates/Acrylamide Copolymer, Aminoethylpropanediol-AMPD-Acrylates/Diacetoneacrylamide Copolymer, Ammonium VA/Acrylates Copolymer, AMPD-Acrylates/Diacetoneacrylamide Copolymer, AMP-Acrylates/Allyl Methacrylate Copolymer, AMP-Acrylates/C1-18 Alkyl Acrylates/C1-8 Alkyl Acrylamide Copolymer, AMP-Acrylates/Diacetoneacrylamide Copolymer, AMP-Acrylates/Dimethylaminoethylmethacrylate Copolymer, Bacillus/Rice Bran Extract/Soybean Extract Ferment Filtrate, Bis-Butyloxyamodimethicone/PEG-60 Copolymer, Butyl Acrylate/Ethylhexyl Methacrylate Copolymer, Butyl Acrylate/Hydroxypropyl Dimethicone Acrylate Copolymer, Butylated PVP, Butyl Ester of Ethylene/MA Copolymer, Butyl Ester of PVM/MA Copolymer, Calcium/Sodium PVM/MA Copolymer, Corn Starch/Acrylamide/ Sodium Acrylate Copolymer, Diethylene Glycolamine/Epichlorohydrin/Piperazine Copolymer, Dimethicone Crosspolymer, Diphenyl Amodimethicone, Ethyl Ester of PVM/MA Copolymer, Hydrolyzed Wheat Protein/PVP Crosspolymer, Isobutylene/Ethylmaleimide/ Hydroxyethylmaleimide Copolymer, Isobutylene/MA Copolymer, Isobutylmethacrylate/Bis-Hydroxypropyl Dimethicone Acrylate Copolymer, Isopropyl Ester of PVM/MA Copolymer, Lauryl Acrylate Crosspolymer, Lauryl Methacrylate/Glycol Dimethacrylate Crosspolymer, MEA-Sulfite, Methacrylic Acid/Sodium Acrylamidomethyl Propane Sulfonate Copolymer, Methacryloyl Ethyl Betaine/Acrylates Copolymer, Octylacrylamide/Acrylates/Butylaminoethyl Methacrylate Copolymer, PEG/PPG-25/25 Dimethicone/Acrylates Copolymer, PEG-8/SMDI Copolymer, Polyacrylamide, Polyacrylate-6, Polybeta-Alanine/Glutaric Acid Crosspolymer, Polybutylene Terephthalate, Polyester-1, Polyethylacrylate, Polyethylene Terephthalate, Polymethacryloyl Ethyl Betaine, Polypentaerythrityl Terephthalate, Polyperfluoroperhydrophenanthrene, Polyquaternium-1, Polyquaternium-2, Polyquaternium-4, Polyquaternium-5, Polyquaternium-6, Polyquaternium-7, Polyquaternium-8, Polyquaternium-9, Polyquaternium-10, Polyquaternium-11, Polyquaternium-12, Polyquaternium-13, Polyquaternium-14, Polyquaternium-15, Polyquaternium-16, Polyquaternium-17, Polyquaternium-18, Polyquaternium-19, Polyquaternium-20, Polyquaternium-22, Polyquaternium-24, Polyquaternium-27, Polyquaternium-28, Polyquaternium-29, Polyquaternium-30, Polyquaternium-31, Polyquaternium-32, Polyquaternium-33, Polyquaternium-34, Polyquaternium-35, Polyquaternium-36, Polyquaternium-37, Polyquaternium-39, Polyquaternium-45, Polyquaternium-46, Polyquaternium-47, Polyquaternium-48, Polyquaternium-49, Polyquaternium-50, Polyquaternium-55, Polyquaternium-56, Polysilicone-9, Polyurethane-1, Polyurethane-6, Polyurethane-10, Polyvinyl Acetate, Polyvinyl Butyral, Polyvinylcaprolactam, Polyvinylformamide, Polyvinyl Imidazolinium Acetate, Polyvinyl Methyl Ether, Potassium Butyl Ester of PVM/MA Copolymer, Potassium Ethyl Ester of PVM/MA Copolymer, PPG-70 Polyglyceryl-10 Ether, PPG-12/SMDI Copolymer, PPG-51/SMDI Copolymer, PPG-10 Sorbitol, PVM/MA Copolymer, PVP, PVP/VA/Itaconic Acid Copolymer, PVP/VA/Vinyl Propionate Copolymer, Rhizobian Gum, Rosin Acrylate, Shellac, Sodium Butyl Ester of PVM/MA Copolymer, Sodium Ethyl Ester of PVM/MA Copolymer, Sodium Polyacrylate, Sterculia Urens Gum, Terephthalic Acid/Isophthalic Acid/Sodium Isophthalic Acid Sulfonate/Glycol Copolymer, Trimethylolpropane Triacrylate, Trimethylsiloxysilylcarbamoyl Pullulan, VA/Crotonates Copolymer, VA/Crotonates/Methacryloxybenzophenone-1 Copolymer, VA/Crotonates/Vinyl Neodecanoate Copolymer, VA/Crotonates/Vinyl Propionate Copolymer, VA/DBM Copolymer, VA/Vinyl Butyl Benzoate/Crotonates Copolymer, Vinylamine/Vinyl Alcohol Copolymer, Vinyl Caprolactam/VP/Dimethylaminoethyl Methacrylate Copolymer, VP/Acrylates/Lauryl Methacrylate Copolymer, VP/Dimethylaminoethylmethacrylate Copolymer, VP/DMAPA Acrylates Copolymer, VP/Hexadecene Copolymer, VP/VA Copolymer, VP/Vinyl Caprolactam/DMAPA Acrylates Copolymer, Yeast Palmitate und Styrene/VP Copolymer.

Insbesondere bevorzugt enthalten erfindungsgemäße Zusammensetzungen zusätzlich wenigstens ein Styrene/VP Copolymer und/oder ein Vinylpyrrolidon-Vinylacetat-Copolymer, vorzugsweise ein Styrene/VP Copolymer.

Die erfindungsgemäßen Zusammensetzungen enthalten des filmbildende Polymere vorzugsweise in Mengen unterhalb 5,0 Gew.-%, bevorzugt in Mengen unterhalb 3,0 Gew.-%, ganz besonders bevorzugt in Mengen zwischen 0,01 und 2,0 Gew.-% und insbesondere in Mengen zwischen 0,04 und 1,0 GEw.-%, jeweils bezogen auf ihr Gesamtgewicht.

Erfindungsgemäß bevorzugte Zusammensetzungen sind dadurch gekennzeichnet, dass mindestens ein Organosiloxan-Oxyalkylen-Copolymer enthalten ist. Derartige Organosiloxan-Oxyalkylen-Copolymere sind beispielsweise unter den Handelsbezeichnung Dow Corning 193 (INCI: PEG-12 Dimethicone) oder Abil B 8843 (INCI: PEG-14 Dimethicone) erhältlich.

Der Gewichtsanteil des Organosiloxan-Oxyalkylen-Copolymers am Gesamtgewicht erfindungsgemäßer Zusammensetzungen beträgt vorzugsweise 0,01 bis 2,0 Gew.-%, bevorzugt 0,02 bis 1,0 Gew.-% und insbesondere 0,05 bis 0,5 Gew.-%

Als weiteren Pflegestoff kann die Zusammensetzung ein Proteinhydrolysat und/oder eines seiner Derivate enthalten. Proteinhydrolysate sind Produktgemische, die durch sauer, basisch oder enzymatisch katalysierten Abbau von Proteinen (Eiweißen) erhalten werden. Unter dem Begriff Proteinhydrolysate werden erfindungsgemäß auch Totalhydrolysate sowie einzelne Aminosäuren und deren Derivate sowie Gemische aus verschiedenen Aminosäuren verstanden. Das Molgewicht der erfindungsgemäß einsetzbaren Proteinhydrolysate liegt zwischen 75, dem Molgewicht für Glycin, und 200.000, bevorzugt beträgt das Molgewicht 75 bis 50.000 und ganz besonders bevorzugt 75 bis 20.000 Dalton.

Als Pflegestoff kann die erfindungsgemäße Zusammensetzung weiterhin mindestens ein Vitamin, ein Provitamin, eine Vitaminvorstufe und/oder eines derer Derivate enthalten. Dabei sind erfindungsgemäß solche Vitamine, Provitamine und Vitaminvorstufen bevorzugt, die üblicherweise den Gruppen A, B, C, E, F und H zugeordnet werden.

Als Pflegestoff kann die erfindungsgemäße Zusammensetzung weiterhin mindestens einen Pflanzenextrakt, aber auch Mono- bzw. Oligosaccharide und/oder Lipide enthalten.

Die erfindungsgemäßen Zusammensetzungen können als Pflegestoff mindestens einen UV-Filter enthalten. Die UV-Filter sind in den erfindungsgemäßen Zusammensetzungen üblicherweise in Mengen 0,01-1 Gew.-%, bezogen auf die erfindungsgemäße pulverförmige Zusammensetzung, enthalten. Mengen von 0,1-0,5 Gew.-% sind bevorzugt.

Einige bevorzugte erfindungsgemäße kosmetische Zusammensetzungen können den folgenden Tabellen entnommen werden (Angaben in Gew.-% bezogen auf das Gesamtgewicht des Reinigungsmittels sofern nicht anders angegeben).

| | Formel 1 | Formel 2 | Formel 3 | Formel 4 | Formel 5 |
|---|---|---|---|---|---|
| Emulgator a) * | 4,0 bis 17 | 6,0 bis 15 | 7,0 bis 14 | 12 | 10 |
| Emulgator b) ** | 6,0 bis 24 | 8,0 bis 22 | 9,0 bis 20 | 12 | 15 |
| Glycerin | 6,0 bis 30 | 10 bis 26 | 12 bis 20 | 18 | 17 |
| Misc | add 100 | add 100 | add 100 | add 100 | add 100 |

| | | | | | |
|---|---|---|---|---|---|
| * Emulgator a) aus der Gruppe der ethoxylierten ungesättigten C16-18 Fettalkohole mit einem durchschnittlichen Ethoxylierungsgrad von 4 bis 6 ** Emulgator b) aus der Gruppe der ethoxylierten gesättigten C16-18 Fettalkohole mit einem durchschnittlichen Ethoxylierungsgrad von 12 bis 30 | | | | | |

| | Formel 6 | Formel 7 | Formel 8 | Formel 9 | Formel 10 |
|---|---|---|---|---|---|
| Oleth-5 | 4,0 bis 17 | 6,0 bis 15 | 7,0 bis 14 | 12 | 10 |
| Emulgator b) ** | 6,0 bis 24 | 8,0 bis 22 | 9,0 bis 20 | 12 | 15 |
| Glycerin | 6,0 bis 30 | 10 bis 26 | 12 bis 20 | 18 | 17 |
| Misc | add 100 | add 100 | add 100 | add 100 | add 100 |

| | | | | | |
|---|---|---|---|---|---|
| ** Emulgator b) aus der Gruppe der ethoxylierten gesättigten C16-18 Fettalkohole mit einem durchschnittlichen Ethoxylierungsgrad von 12 bis 30 | | | | | |

| | Formel 11 | Formel12 | Formel 13 | Formel 14 | Formel 15 |
|---|---|---|---|---|---|
| Emulgator a) * | 4,0 bis 17 | 6,0 bis 15 | 7,0 bis 14 | 12 | 10 |
| Ceteth-20 | 6,0 bis 24 | 8,0 bis 22 | 9,0 bis 20 | 12 | 15 |
| Glycerin | 6,0 bis 30 | 10 bis 26 | 12 bis 20 | 18 | 17 |
| Misc | add 100 | add 100 | add 100 | add 100 | add 100 |

| | | | | | |
|---|---|---|---|---|---|
| * Emulgator a) aus der Gruppe der ethoxylierten ungesättigten C16-18 Fettalkohole mit einem durchschnittlichen Ethoxylierungsgrad von 4 bis 6 | | | | | |

| | Formel 16 | Formel 17 | Formel 18 | Formel 19 | Formel 20 |
|---|---|---|---|---|---|
| Oleth-5 | 4,0 bis 17 | 6,0 bis 15 | 7,0 bis 14 | 12 | 10 |
| Ceteth-20 | 6,0 bis 24 | 8,0 bis 22 | 9,0 bis 20 | 12 | 15 |
| Glycerin | 6,0 bis 30 | 10 bis 26 | 12 bis 20 | 18 | 17 |
| Misc | add 100 | add 100 | add 100 | add 100 | add 100 |

| | Formel 21 | Formel 22 | Formel 23 | Formel 24 | Formel 25 |
|---|---|---|---|---|---|
| Emulgator a) * | 4,0 bis 17 | 6,0 bis 15 | 7,0 bis 14 | 12 | 10 |
| Emulgator b) ** | 6,0 bis 24 | 8,0 bis 22 | 9,0 bis 20 | 12 | 15 |
| Glycerin | 6,0 bis 30 | 10 bis 26 | 12 bis 20 | 18 | 17 |
| Fettstoff c) *** | 6,0 bis 22 | 8,0 bis 20 | 10 bis 28 | 12 | 14 |
| Misc | add 100 | add 100 | add 100 | add 100 | add 100 |

| | | | | | |
|---|---|---|---|---|---|
| * Emulgator a) aus der Gruppe der ethoxylierten ungesättigten C16-18 Fettalkohole mit einem durchschnittlichen Ethoxylierungsgrad von 4 bis 6 ** Emulgator b) aus der Gruppe der ethoxylierten gesättigten C16-18 Fettalkohole mit einem durchschnittlichen Ethoxylierungsgrad von 12 bis 30 *** Fettstoff c) aus der Gruppe der mineralischen, natürlichen und synthetischen Öle | | | | | |

| | Formel 26 | Formel 27 | Formel 28 | Formel 29 | Formel 30 |
|---|---|---|---|---|---|
| Oleth-5 | 4,0 bis 17 | 6,0 bis 15 | 7,0 bis 14 | 12 | 10 |
| Emulgator b) ** | 6,0 bis 24 | 8,0 bis 22 | 9,0 bis 20 | 12 | 15 |
| Glycerin | 6,0 bis 30 | 10 bis 26 | 12 bis 20 | 18 | 17 |
| Fettstoff c) *** | 6,0 bis 22 | 8,0 bis 20 | 10 bis 28 | 12 | 14 |
| Misc | add 100 | add 100 | add 100 | add 100 | add 100 |

| | | | | | |
|---|---|---|---|---|---|
| ** Emulgator b) aus der Gruppe der ethoxylierten gesättigten C16-18 Fettalkohole mit einem durchschnittlichen Ethoxylierungsgrad von 12 bis 30 *** Fettstoff c) aus der Gruppe der mineralischen, natürlichen und synthetischen Öle | | | | | |

| | Formel 31 | Formel 32 | Formel 33 | Formel 34 | Formel 35 |
|---|---|---|---|---|---|
| Emulgator a) * | 4,0 bis 17 | 6,0 bis 15 | 7,0 bis 14 | 12 | 10 |
| Ceteth-20 | 6,0 bis 24 | 8,0 bis 22 | 9,0 bis 20 | 12 | 15 |
| Glycerin | 6,0 bis 30 | 10 bis 26 | 12 bis 20 | 18 | 17 |
| Fettstoff c) *** | 6,0 bis 22 | 8,0 bis 20 | 10 bis 28 | 12 | 14 |
| Misc | add 100 | add 100 | add 100 | add 100 | add 100 |

| | | | | | |
|---|---|---|---|---|---|
| * Emulgator a) aus der Gruppe der ethoxylierten ungesättigten C16-18 Fettalkohole mit einem durchschnittlichen Ethoxylierungsgrad von 4 bis 6 *** Fettstoff c) aus der Gruppe der mineralischen, natürlichen und synthetischen Öle | | | | | |

| | Formel 36 | Formel 37 | Formel 38 | Formel 39 | Formel 40 |
|---|---|---|---|---|---|
| Oleth-5 | 4,0 bis 17 | 6,0 bis 15 | 7,0 bis 14 | 12 | 10 |
| Ceteth-20 | 6,0 bis 24 | 8,0 bis 22 | 9,0 bis 20 | 12 | 15 |
| Glycerin | 6,0 bis 30 | 10 bis 26 | 12 bis 20 | 18 | 17 |
| Fettstoff c) *** | 6,0 bis 22 | 8,0 bis 20 | 10 bis 28 | 12 | 14 |
| Misc | add 100 | add 100 | add 100 | add 100 | add 100 |

| | | | | | |
|---|---|---|---|---|---|
| *** Fettstoff c) aus der Gruppe der mineralischen, natürlichen und synthetischen Öle | | | | | |

| | Formel 41 | Formel 42 | Formel 43 | Formel 44 | Formel 45 |
|---|---|---|---|---|---|
| Oleth-5 | 4,0 bis 17 | 6,0 bis 15 | 7,0 bis 14 | 12 | 10 |
| Ceteth-20 | 6,0 bis 24 | 8,0 bis 22 | 9,0 bis 20 | 12 | 15 |
| Glycerin | 6,0 bis 30 | 10 bis 26 | 12 bis 20 | 18 | 17 |
| Paraffinum Liquidum | 6,0 bis 22 | 8,0 bis 20 | 10 bis 28 | 12 | 14 |
| Misc | add 100 | add 100 | add 100 | add 100 | add 100 |

| | Formel 46 | Formel 47 | Formel 48 | Formel 49 | Formel 50 |
|---|---|---|---|---|---|
| Emulgator a) * | 4,0 bis 17 | 6,0 bis 15 | 7,0 bis 14 | 12 | 10 |
| Emulgator b) ** | 6,0 bis 24 | 8,0 bis 22 | 9,0 bis 20 | 12 | 15 |
| Glycerin | 6,0 bis 30 | 10 bis 26 | 12 bis 20 | 18 | 17 |
| Fettstoffs)*** | 6,0 bis 22 | 8,0 bis 20 | 10 bis 28 | 12 | 14 |
| Wasser | 20 bis 70 | 30 bis 60 | 40 bis 55 | 45 | 43 |
| Misc | add 100 | add 100 | add 100 | add 100 | add 100 |

| | | | | | |
|---|---|---|---|---|---|
| * Emulgator a) aus der Gruppe der ethoxylierten ungesättigten C16-18 Fettalkohole mit einem durchschnittlichen Ethoxylierungsgrad von 4 bis 6 ** Emulgator b) aus der Gruppe der ethoxylierten gesättigten C16-18 Fettalkohole mit einem durchschnittlichen Ethoxylierungsgrad von 12 bis 30 *** Fettstoff c) aus der Gruppe der mineralischen, natürlichen und synthetischen Öle | | | | | |

| | Formel 51 | Formel 52 | Formel 53 | Formel 54 | Formel 55 |
|---|---|---|---|---|---|
| Oleth-5 | 4,0 bis 17 | 6,0 bis 15 | 7,0 bis 14 | 12 | 10 |
| Emulgator b) ** | 6,0 bis 24 | 8,0 bis 22 | 9,0 bis 20 | 12 | 15 |
| Glycerin | 6,0 bis 30 | 10 bis 26 | 12 bis 20 | 18 | 17 |
| Fettstoff c) *** | 6,0 bis 22 | 8,0 bis 20 | 10 bis 28 | 12 | 14 |
| Wasser | 20 bis 70 | 30 bis 60 | 40 bis 55 | 45 | 43 |
| Misc | add 100 | add 100 | add 100 | add 100 | add 100 |

| | | | | | |
|---|---|---|---|---|---|
| ** Emulgator b) aus der Gruppe der ethoxylierten gesättigten C16-18 Fettalkohole mit einem durchschnittlichen Ethoxylierungsgrad von 12 bis 30 *** Fettstoff c) aus der Gruppe der mineralischen, natürlichen und synthetischen Öle | | | | | |

| | Formel 56 | Formel 57 | Formel 58 | Formel 59 | Formel 60 |
|---|---|---|---|---|---|
| Emulgator a) * | 4,0 bis 17 | 6,0 bis 15 | 7,0 bis 14 | 12 | 10 |
| Ceteth-20 | 6,0 bis 24 | 8,0 bis 22 | 9,0 bis 20 | 12 | 15 |
| Glycerin | 6,0 bis 30 | 10 bis 26 | 12 bis 20 | 18 | 17 |
| Fettstoff c) *** | 6,0 bis 22 | 8,0 bis 20 | 10 bis 28 | 12 | 14 |
| Wasser | 20 bis 70 | 30 bis 60 | 40 bis 55 | 45 | 43 |
| Misc | add 100 | add 100 | add 100 | add 100 | add 100 |

| | | | | | |
|---|---|---|---|---|---|
| * Emulgator a) aus der Gruppe der ethoxylierten ungesättigten C16-18 Fettalkohole mit einem durchschnittlichen Ethoxylierungsgrad von 4 bis 6 *** Fettstoff c) aus der Gruppe der mineralischen, natürlichen und synthetischen Öle | | | | | |

| | Formel 61 | Formel 62 | Formel 63 | Formel 64 | Formel 65 |
|---|---|---|---|---|---|
| Oleth-5 | 4,0 bis 17 | 6,0 bis 15 | 7,0 bis 14 | 12 | 10 |
| Ceteth-20 | 6,0 bis 24 | 8,0 bis 22 | 9,0 bis 20 | 12 | 15 |
| Glycerin | 6,0 bis 30 | 10 bis 26 | 12 bis 20 | 18 | 17 |
| Fettstoff c) *** | 6,0 bis 22 | 8,0 bis 20 | 10 bis 28 | 12 | 14 |
| Wasser | 20 bis 70 | 30 bis 60 | 40 bis 55 | 45 | 43 |
| Misc | add 100 | add 100 | add 100 | add 100 | add 100 |

| | | | | | |
|---|---|---|---|---|---|
| *** Fettstoff c) aus der Gruppe der mineralischen, natürlichen und synthetischen Öle | | | | | |

| | Formel 66 | Formel 67 | Formel 68 | Formel 69 | Formel 70 |
|---|---|---|---|---|---|
| Oleth-5 | 4,0 bis 17 | 6,0 bis 15 | 7,0 bis 14 | 12 | 10 |
| Ceteth-20 | 6,0 bis 24 | 8,0 bis 22 | 9,0 bis 20 | 12 | 15 |
| Glycerin | 6,0 bis 30 | 10 bis 26 | 12 bis 20 | 18 | 17 |
| Paraffinum Liquidum | 6,0 bis 22 | 8,0 bis 20 | 10 bis 28 | 12 | 14 |
| Wasser | 20 bis 70 | 30 bis 60 | 40 bis 55 | 45 | 43 |
| Misc | add 100 | add 100 | add 100 | add 100 | add 100 |

Besonders bevorzugte erfindungsgemäße kosmetische Zusammensetzungen liegen in Form einer Mikroemulsion vor.

Bevorzugte erfindungsgemäße kosmetische Zubereitungen sind transparent. Als "transparent" werden solche Zubereitungen bezeichnet, die eine Trübung ≤ 250 NTU (Nephelometric Turbidity Unit = Nephelometrischer Trübungswert), bevorzugt eine Trübung ≤ 150 NTU, besonders bevorzugt eine Trübung ≤ 100 NTU, insbesndere eine Trübung ≤ 50 NTU und ganz besonders bevorzugt ≤ 20 NTU aufweisen. Die Bestimmung des Trübungswert kann beispielsweise mit dem Turbidimeter 2100P der Firma Hach erfolgen.

Besonders bevorzugte erfindungsgemäße kosmetische Zusammensetzungen liegen in Form von Mikroemulsionen vor, die eine Trübung ≤ 250 NTU (Nephelometric Turbidity Unit = Nephelometrischer Trübungswert), bevorzugt eine Trübung ≤ 150 NTU, besonders bevorzugt eine Trübung ≤ 100 NTU, insbesndere eine Trübung ≤ 50 NTU und ganz besonders bevorzugt ≤ 20 NTU aufweisen. Die Bestimmung des Trübungswert kann beispielsweise mit dem Turbidimeter 2100P der Firma Hach erfolgen.

Bevorzugte erfindungsgemäße kosmetische Zubereitungen weisen eine Härte zwischen 0,05 und 0,4 mm, vorzugsweise zwischen 0,1 und 0,2 mm, gemessen mit einem Textur-Analysator, auf. Kosmetische Zubereitungen mit einer entsprechenden Härte lassen sich ein einfacher Weise auf den keratinischen Fasern verteilen.
Die Messung der Härte der kosmetischen Zubereitung mittels des Textur-Analsysators erfolgt bei 21°C und 50% relativer Luftfeuchte in einem Probevolumen von 75 ml und einer Probenhöhe von 3 cm. Der zylindrische Probekörper (7mm Durchmesser) wird mit einer Testgeschwindigkeit von 1 mm/sec in die Probe vorgetrieben. Der Kraftgrenzwert, bei welchem die Datenerhebung beginnt, beträgt 3 g. Bei einem Kraftgrenzwert von 23 g stoppt der Vortrieb. Die Härte der kosmetischen Zubereitung ergibt sich als Differenz der Eindringtiefe des Probekörpers in die Zubereitung bei einer Kraft von 23 g abzüglich der Eindringtiefe des Probekörpers in die Zubereitung bei einer Kraft von 3 g.

Wie eingangs ausgeführt, eigen sich die erfindungsgemäßen kosmetischen Zusammensetzungen insbesondere zur Verformung keratinischer Fasern. Ein weiterer Gegenstand dieser Anmeldung ist daher die Verwendung einer kosmetischen Zusammensetzung enthaltend
a) 4,0 bis 17 Gew.-%mindestens eines Emulgators a) aus der Gruppe der ethoxylierten ungesättigten C16-18 Fettalkohole mit einem durchschnittlichen Ethoxylierungsgrad von 4 bis 6;
b) 6,0 bis 24 Gew.-% mindestens eines Emulgators b) aus der Gruppe der ethoxylierten gesättigten C16-18 Fettalkohole mit einem durchschnittlichen Ethoxylierungsgrad von 12 bis 30;
c) 6,0 bis 30 Gew.-% Glycerin,
zur temporären Verformung keratinhaltiger Fasern, insbesondere menschlicher Haare.

Die temporäre Verformung der keratinischen Fasern geht mit einer Verbesserung des Griffs und des Glanzes der Fasern einher. Die Verbesserung von Griff und Glanz ist dabei nicht nur im Vergleich zu unbehandeltem Haar sondern auch im Vergleich mit kosmetischen Zusammensetzungen des Standes der Technik nachweisbar. Ein weiterer Gegenstand dieser Anmeldung ist daher die Verwendung einer kosmetischen Zusammensetzung enthaltend
a) 4,0 bis 17 Gew.-%mindestens eines Emulgators a) aus der Gruppe der ethoxylierten ungesättigten C16-18 Fettalkohole mit einem durchschnittlichen Ethoxylierungsgrad von 4 bis 6;
b) 6,0 bis 24 Gew.-% mindestens eines Emulgators b) aus der Gruppe der ethoxylierten gesättigten C16-18 Fettalkohole mit einem durchschnittlichen Ethoxylierungsgrad von 12 bis 30;
c) 6,0 bis 30 Gew.-% Glycerin,
zur Verbesserung des Griffs und/oder des Glanzes keratinhaltiger Fasern, insbesondere menschlicher Haare.

## Patentansprüche

1. Verwendung einer kosmetischen Zusammensetzung, enthaltend
a) 4,0 bis 17 Gew.-% mindestens eines Emulgators a) aus der Gruppe der ethoxylierten ungesättigten C16-18 Fettalkohole mit einem durchschnittlichen Ethoxylierungsgrad von 4 bis 6;
b) 6,0 bis 24 Gew.-% mindestens eines Emulgators b) aus der Gruppe der ethoxylierten gesättigten C16-18 Fettalkohole mit einem durchschnittlichen Ethoxylierungsgrad von 12 bis 30;
c) 6,0 bis 30 Gew.-% Glycerin
zur Verbesserung des Griffs menschlicher Haare.

2. Verwendung nach Anspruch 1, **dadurch gekennzeichnet, dass** der Emulgator a) einen durchschnittlichen Ethoxilierungsgrad von 5 aufweist.

3. Verwendung nach einem der vorherigen Ansprüche, **dadurch gekennzeichnet, dass** die Zusammensetzung bezogen auf ihr Gesamtgewicht 6,0 bis 15 Gew.-%, vorzugsweise 8,0 bis 13 Gew.-% des Emulgators a) enthält.

4. Verwendung nach einem der vorherigen Ansprüche, dadurch gekenntzeichnet, dass der Emulgator b) einen durchschnittlichen Ethoxilierungsgrad von 15 bis 25, vorzugsweise von 20 aufweist.

5. Verwendung nach einem der vorherigen Ansprüche, dadurch gekenntzeichnet, dass die Zusammensetzung bezogen auf ihr Gesamtgewicht 8,0 bis 22 Gew.-% und insbesondere 10 bis 18 Gew.-% des Emulgators b) enthält.

6. Verwendung nach einem der vorherigen Ansprüche, dadurch gekenntzeichnet, dass die Zusammensetzung bezogen auf ihr Gesamtgewicht 10 bis 26 Gew.-%, vorzugsweise 12 bis 20 Gew.-% Glycerin enthält.

7. Verwendung nach einem der vorherigen Ansprüche, dadurch gekenntzeichnet, dass **dadurch gekennzeichnet, dass** das Gewichtsverhältnis des Emulgators a) zum Emulgator b) 2:1 bis 1:5, vorzugsweise 1:1 bis 1:2 beträgt.

8. Verwendung nach einem der vorherigen Ansprüche, dadurch gekenntzeichnet, dass die Zusammensetzung bezogen auf ihr Gesamtgewicht 20 bis 70 Gew.-%, vorzugsweise 30 bis 60 Gew.-% und insbesondere 40 bis 55 Gew.-% Wasser enthält.

9. Verwendung nach einem der vorherigen Ansprüche, **dadurch gekennzeichnet, dass** die Zusammensetzung bezogen auf ihr Gesamtgewicht 6,0 bis 22 Gew.-%, vorzugsweise 8,0 bis 20 Gew.-% und insbesondere 10 bis 18 Gew.-% eines Fettstoffs c), aus der Gruppe der mineralischen, natürlichen und synthetischen Öle, insbesondere bevorzugt aus der Gruppe der mineralischen Öle, enthält.

## Claims

1. The use of a cosmetic composition containing
a) from 4.0 to 17 wt.% at least one emulsifier a) from the group of the ethoxylated unsaturated C16-18 fatty alcohols having an average degree of ethoxylation of from 4 to 6;
b) from 6.0 to 24 wt.% at least one emulsifier b) from the group of the ethoxylated saturated C16-18 fatty alcohols having an average degree of ethoxylation of from 12 to 30;
c) from 6.0 to 30 wt.% glycerol
for improving the feel of human hair.

2. The use according to claim 1, **characterized in that** emulsifier a) has an average degree of ethoxylation of 5.

3. The use according to either of the preceding claims, **characterized in that** the composition contains, based on its total weight, from 6.0 to 15 wt.%, preferably from 8.0 to 13 wt.%, emulsifier a).

4. The use according to one of the preceding claims, **characterized in that** emulsifier b) has an average degree of ethoxylation of from 15 to 25, preferably of 20.

5. The use according to one of the preceding claims, **characterized in that** the composition contains, based on its total weight, from 8.0 to 22 wt.% and in particular from 10 to 18 wt.% emulsifier b).

6. The use according to one of the preceding claims, **characterized in that** the composition contains, based on its total weight, from 10 to 26 wt.%, preferably from 12 to 20 wt.%, glycerol.

7. The use according to one of the preceding claims, **characterized in that** the weight ratio of emulsifier a) to emulsifier b) is from 2:1 to 1:5, preferably from 1:1 to 1:2.

8. The use according to one of the preceding claims, **characterized in that** the composition contains, based on its total weight, from 20 to 70 wt.%, preferably from 30 to 60 wt.% and in particular from 40 to 55 wt.%, water.

9. The use according to one of the preceding claims, **characterized in that** the composition contains, based on its total weight, from 6.0 to 22 wt.%, preferably from 8.0 to 20 wt.% and in particular from 10 to 18 wt.%, a fatty substance c) from the group of the mineral, natural and synthetic oils, particularly preferably from the group of the mineral oils.

## Revendications

1. Utilisation d'une composition cosmétique contenant
a) de 4,0 à 17% en poids d'au moins un émulsifiant a) du groupe des alcools gras en C16 à C18 insaturés éthoxylés ayant un degré d'éthoxylation moyen de 4 à 6 ;
b) de 6,0 à 24% en poids d'au moins un émulsifiant b) du groupe des alcools gras en C16 à C18 saturés éthoxylés ayant un degré d'éthoxylation moyen de 12 à 30 ;
c) de 6,0 à 30% en poids de glycérol
pour améliorer le toucher de cheveux humains.

2. Utilisation selon la revendication 1, **caractérisée en ce que** l'émulsifiant a) a un degré d'éthoxylation moyen de 5.

3. Utilisation selon l'une des revendications précédentes, **caractérisée en ce que** la composition contient, sur la base de son poids total, de 6,0 à 15% en poids, de préférence de 8,0 à 13% en poids, de l'émulsifiant a).

4. Utilisation selon l'une des revendications précédentes, **caractérisée en ce que** l'émulsifiant b) a un degré d'éthoxylation moyen de 15 à 25, de préférence de 20.

5. Utilisation selon l'une des revendications précédentes, **caractérisée en ce que** la composition contient, sur la base de son poids total, de 8,0 à 22% en poids et en particulier de 10 à 18% en poids de l'émulsifiant b).

6. Utilisation selon l'une des revendications précédentes, **caractérisée en ce que** la composition contient, sur la base de son poids total, de 10 à 26% en poids, de préférence de 12 à 20% en poids, de glycérol.

7. Utilisation selon l'une des revendications précédentes, **caractérisée en ce que** le rapport en poids de l'émulsifiant a) à l'émulsifiant b) est de 2:1 à 1:5, de préférence de 1:1 à 1:2.

8. Utilisation selon l'une des revendications précédentes, **caractérisée en ce que** la composition contient, sur la base de son poids total, de 20 à 70% en poids, de préférence de 30 à 60% en poids et en particulier de 40 à 55% en poids, d'eau.

9. Utilisation selon l'une des revendications précédentes, **caractérisée en ce que** la composition contient, sur la base de son poids total, de 6,0 à 22% en poids, de préférence de 8,0 à 20% en poids et en particulier de 10 à 18% en poids, d'un corps gras c) du groupe des huiles minérales, naturelles et synthétiques, de façon particulièrement préférée du groupe des huiles minérales.
